# EUROPÄISCHE PATENTSCHRIFT

(11) **EP 2 102 103 B1**
(45) Veröffentlichungstag und Bekanntmachung des Hinweises auf die Patenterteilung: **21.01.2015**
(21) Anmeldenummer: 07819639.1
(22) Anmeldetag: 07.11.2007
(51) Int. Cl.: C01B 25/42, C12Q 1/66, G01N 21/76, G01N 33/84

(54) **VERFAHREN ZUM NACHWEIS VON PYROPHOSPHAT MIT BIOLUMINESZENZ DETEKTION**
METHOD FOR DETECTING PYROPHOSPHATE BY MEANS OF BIOLUMINESCENCE DETECTION
PROCÉDÉ POUR DÉCELER DU PYROPHOSPHATE PAR DÉTECTION DE BIOLUMINESCENCE

(30) Priorität: 20.11.2006 DE 102006054562
(43) Veröffentlichungstag der Anmeldung: 23.09.2009
(73) Patentinhaber: Bayer Intellectual Property GmbH, 40789 Monheim (DE)
(72) Erfinder: BURKHARDT, Nils, 42553 Velbert (DE); HEITMEIER, Stefan, 42489 Wülfrath (DE)
(74) Vertreter: BIP Patents
(86) Internationale Anmeldenummer: PCT/EP2007/009625
(87) Internationale Veröffentlichungsnummer: WO 2008/061626

(56) Entgegenhaltungen:
- WO-A-2006/013837
- DE-A1- 19 602 662
- MCELROY W D ET AL: "The mechanism of action of pyrophosphate in firefly luminescence" ARCHIVES OF BIOCHEMISTRY AND BIOPHYSICS, NEW YORK, US, US, Bd. 46, Nr. 2, Oktober 1953 (1953-10), Seiten 399-416, XP002991520 ISSN: 0003-9861
- AHMAD, MUSHTAG ET AL: "Analytical aspects of the firefly luciferase reaction kinetics" BIOLUMIN. CHEMILUMIN., [INT. SYMP. ANAL. APPL. BIOLUMIN. CHEMILUMIN.], 2ND , MEETING DATE 1980, 435-41. EDITOR(S): DELUCA, MARLENE A.; MCELROY, WILLIAM DAVID. PUBLISHER: ACADEMIC, NEW YORK, N. Y. CODEN: 45UJAC, 1981, XP001537875
- RONAGHI M ET AL: "REAL-TIME DNA SEQUENCING USING DETECTION OF PYROSPHOSPHATE RELEASE" ANALYTICAL BIOCHEMISTRY, ACADEMIC PRESS, SAN DIEGO, CA, US, Bd. 242, November 1996 (1996-11), Seiten 84-89, XP002055379 ISSN: 0003-2697

## Beschreibung

Die Erfindung betrifft Verfahren zum Nachweis von Pyrophosphat mit Biolumineszenz Detektion. Darüber hinaus werden Verfahren zur Messung von chemischen, insbesondere Enzym katalysierten Reaktionen beschrieben, bei denen Pyrophosphat gebildet oder verbraucht wird. Solche Reaktionen werden beispielsweise von Guanylylcyclasen, Adenylylcyclasen, DNA- oder RNA-Polymerasen katalysiert.

Die neuen Verfahren zeichnen sich durch hohe Sensitivität und geringe Störanfälligkeit aus, lassen sich leicht automatisieren und miniaturisieren und eignen sich zudem für die Durchführung kontinuierlicher Messungen. Besonders vorteilhaft können die Verfahren im Bereich der medizinischen Diagnostik und der biomedizinischen Forschung, einschließlich der pharmazeutischen Wirkstoffforschung, eingesetzt werden.

Pyrophosphat (Diphosphate(V), PPi) ist ein zentraler Metabolit des Zellstoffwechsels, der bei zahlreichen enzymatischen Reaktionen gebildet oder verbraucht wird; so erfolgen beispielsweise zentrale Stoffwechselreaktionen, wie die Synthese von Polysacchariden, Proteinen und Nukleinsäuren unter Bildung von Pyrophosphat. Pyrophosphat kommt darüber hinaus auch in der unbelebten Umwelt vor und wird bei verschiedenen technischen Reaktionen gebildet oder verbraucht.

Verfahren zum Nachweis und zur Quantifizierung von Pyrophosphat können daher in verschiedenen Bereichen eingesetzt werden; von besonderem Interesse ist die Verwendung im Bereich der medizinischen Diagnostik, der biomedizinischen Forschung, insbesondere der Wirkstoffforschung und der Arzneimittelforschung, sowie im Bereich der Umwelt- und Lebensmittelanalytik. Dabei sind neben der Analyse von Pyrophosphatgehalten vor allem die Messung von chemischen Reaktionen, bei denen Pyrophosphat gebildet oder verbraucht wird, sowie die Bestimmung von Enzymaktivitäten, die solche Reaktionen katalysieren, von Bedeutung.

Im Bereich der medizinischen Diagnostik bietet sich der Einsatz von Verfahren zum Nachweis von Pyrophosphat überall dort an, wo der Pyrophosphatgehalt der Testprobe ein Indikator für eine krankhafte Veränderung sein kann. Besonders interessant können Proben von Körperflüssigkeiten oder Aufarbeitungen von Gewebe sein, zum Beispiel Proben von Blut, Serum, Urin, Rückenmarksflüssigkeit, Gelenkflüssigkeit oder Aufarbeitungen daraus. Verfahren zur Analyse derartiger Testproben sollen sich durch möglichst hohe Sensitivität und geringe Störanfälligkeit, insbesondere gegenüber typischen Probenbestandteilen, wie beispielsweise Phosphat oder ATP, auszeichnen. Darüber hinaus ist es für eine effiziente und kostengünstige Durchführung der Analysen wünschenswert, daß sich die Verfahren leicht automatisieren und miniaturisieren lassen.

Im Bereich der biomedizinischen Forschung, insbesondere der pharmazeutischen Wirkstoffforschung werden zur Auffindung neuer Wirkstoffkandidaten regelmäßig große Substanzbibliotheken mit teilweise mehr als einer Million Substanzen mit Hilfe automatisierter Verfahren durchgemustert (Hochdurchsatz Screening; High Throughput Screening); dabei richtet sich das Screening typischerweise auf die Identifizierung von Modulatoren einer biologischen Aktivität, beispielsweise einer Enyzmaktivität, welche das Ziel einer medizinischen Therapie sein könnte. Enyzme, welche Pyrophosphat bildende oder verbrauchende Reaktionen katalysieren und Ziel einer medizinischen Therapie sein könnten, sind zum Beispiel Guanylylcyclasen, Adenylylcyclasen, DNA- und RNA- Polymerasen sowie Squalensynthase. Verfahren zur Messung von solchen Enzymaktivitäten im pharmazeutischen Hochdurchsatz Screening sollen sich leicht automatisieren und zur Begrenzung der Kosten für Reagenzien und Testsubstanzen auch miniaturisieren lassen. Darüber hinaus ist zum Nachweis auch geringer Enzymaktivitäten eine möglichst hohe Sensitivität und zur Erreichung einer hohen Datenqualität eine möglichst geringe Störanfälligkeit wünschenswert.

Zur weiteren enzymkinetischen Charakterisierung der Modulatoren, einschließlich der Bestimmung des Inhibitionsmechanismus, der Bildungs- und Dissoziationsgeschwindigkeit des Enyzm-Modulator-Komplexes oder der mit der Bildung und Dissoziation des Enyzm-Modulator-Komplexes verbundenen Energieumsätze, wird typischerweise der Verlauf der untersuchten Enyzmreaktionen aufgezeichnet und ausgewertet. Geeignete Verfahren sollen daher den Verlauf der Reaktion mit möglichst hoher zeitlicher Auflösung, idealerweise kontinuierlich, abbilden.

Eine kontinuierliche Messung der Pyrophosphatkonzentration kann auch bei Anwendungen im Bereich der Genomanalyse und der Genexpressionsanalyse wünschenswert sein, zum Beispiel bei der Nukleinsäuresequenzierung (Pyrosequencing) oder der Messung von Nukleinsäureamplifikationen durch Polymerase Kettenreaktion (Quantitative PCR). Dabei zeichnen sich geeignete Verfahren zusätzlich durch eine möglichst geringe Störanfäligkeit gegenüber Phosphat und ATP aus.

Biolumineszenz ist ein in der Natur weit verbreitetes, aber auch technisch genutztes Phänomen und bezeichnet die Emission von Licht im Verlauf einer enzymkatalysierten chemischen Reaktion; bioluminogene Reaktionen werden beispielsweise durch Luziferasen oder Photoproteine katalysiert. Seit langem bekannt ist die durch *Photinus pyralis* Luziferase katalysierte oxidative Decarboxylierung von D-Luziferin zu Oxyluziferin in Gegenwart von Adenosintriphosphat (ATP), Mg²⁺ und Sauerstoff (O₂). Untersuchungen zur Charakterisierung dieser Reaktion sowie des Reaktionsmechanismus werden zum Beispiel in McElroy, Proc. Natl. Acad. Sci. USA 33 (1947) 342-345, McElroy, J. Biol. Chem. 191 (1951) 547-557, McElroy et al., Arch. Biochem. Biophys. 46 (1953) 399-416, Green & McElroy, Biochim. Biophys. Acta 20 (1956) 170-176, Bitler & McElroy, Arch. Biochem. Biophys. 72 (1957) 358-368, Rhodes et al., J. Biol. Chem. 233 (1958) 1528-1537, White et al., J. Am. Chem. Soc. 85 (1963) 337-343, Denburg et al., Arch. Biochem. Biophys. 134 (1969) 381-394, Lee et al., Arch. Biochem. Biophys. 141 (1970) 38-52, Denburg & McElroy, Arch. Biochem. Biophys. 141 (1970) 668-675, Lundin, (1993) in: Biolum. Chemilum.: Status Report (Ed. Szalay, Kricka), 291-295, Fontes et al., Biochem. Biophys. Res. Comun. 237 (1997) 445-450, Fontes et al., FEBS Lett. 438 (1998) 190-194, Sillero et al., Pharmacol. Therap. 87 (2000) 91-102, WO 9640988 und Eriksson et al., Anal. Biochem. 293 (2001) 67-70 genauer dargelegt; dabei werden sowohl aktivatorische als auch inhibitorische Effekte von Pyrophosphat beschrieben. Das bei der Reaktion emittierte Lumineszenzlicht kann sehr empfindlich vermessen und dadurch der Verlauf der Reaktion genau verfolgt werden. Die durch *Photinus pyralis* Luziferase katalysierte Reaktion wird üblicherweise für den Nachweis von ATP, aber auch zur Messung von Enzymreaktionen, bei denen ATP gebildet oder verbraucht wird, eingesetzt (Lundin (1982), in Luminescent Assays: Perspectives in Endocrinology and Clin. Chem. (Ed. Serio, Pazzagli) 29-45). Das Gen der *Photinus pyralis* Luziferase wird darüber hinaus häufig als Reportergen in zellulären Testsystemen verwendet (Gould et al., Anal. Biochem. 175 (1988) 5-13).

Es ist bekannt, dass alle Käferluziferasen (Luziferasen isoliert aus den Superfamilien Elateroidea und Cantharoidea) eine bioluminogene Reaktion katalysieren, die dieselben Substrate verbraucht, Adenosintriphopsphat (ATP), Luziferin und molekularen Sauerstoff. Es ist anzunehmen, dass alle Käferluziferasen denselben Reaktionsmechanismus verwenden (US 6.387.675 B1).

Im Stand der Technik sind verschiedene Verfahren zum Nachweis von Pyrophosphat bekannt.

Flynn et al., J. Biol. Chem. 211 (1954) 791-796, Grindey et al., Anal. Biochem. 33 (1970) 114-119, Putnins et al., Anal. Biochem. 68 (1975) 185-195, Heinonen et al., Anal. Biochem. 117 (1981) 293-300, und Mansurova et al., Anal. Biochem. 176 (1991) 390-94 beschreiben direkte chemische Nachweisverfahren für Pyrophosphat. Bei den beschriebenen Verfahren wird die Pyrophosphat enthaltende Testprobe mit einer sauren Molybdatlösung und einem reduzierenden Thiolreagenz (Cystein, Bisulfid/Thioglycerol oder Mercaptoethanol) versetzt und die entstehenden blauen Komplexe UV-spektrometrisch quantifiziert. Problematisch ist die Störanfälligkeit dieser Nachweisverfahren, insbesondere gegenüber Phosphat. Darüber hinaus ist die langsame Komplexbildung (mit Cystein >10-15 min) für eine effiziente automatisierte Durchführung von Nachteil.

Heinonen et al., Anal. Biochem. 59 (1974) 366-374, und Russel et al., J. Clin. Invest. 50 (1971) 961-969, beschreiben radioaktive Nachweisverfahren, welche mehrere aufwendige Arbeitschritte umfassen und sich daher nur schwer automatisieren lassen. Darüber hinaus sind diese Verfahren nicht geeignet für kontinuierliche Messungen zur Bestimmung des zeitlichen Verlaufs einer Pyrophosphat Probenkonzentration.

Im Stand der Technik sind außerdem Pyrophosphat Nachweise mit gekoppelten Enzymreaktionen bekannt; bei diesen Verfahren wird Pyrophosphat enzymatisch umgesetzt und als Reaktionsprodukt von einer oder von mehreren gekoppelten Enzymreaktionen nachgewiesen.

Ein solches Verfahren wird zum Beispiel von Johnson et al., Anal. Biochem. 26 (1968) 137-145, beschrieben; dabei wird Pyrophosphat mit UDP-Glucose unter Katalyse von Glucosepyrophosphatase zu UTP und Glucose-1-P umgesetzt; das gebildete Glucose-1-P isomerisiert unter Katalyse von Phosphoglucomutase zu Glucose-6-P, welches unter Verbrauch von NADP und katalysiert durch Glucose-6-P-Dehydrogenase zu Gluconolacton-6-P abreagiert. Das dabei gebildete NADPH wird durch UV-Absorptionsmessung quantifiziert und zur Bestimmung des Pyrophosphatgehalts der Testprobe herangezogen. Weitere Nachweisverfahren für Pyrophosphat mit gekoppelten Enzymreaktionen werden beispielsweise von Cartier et al., Anal. Biochem. 61 (1974) 416-428, Cheung et al., Anal.Biochem. 83 (1977) 61-63, Reeves et al., Anal. Biochem. 28 (1969) 282-287, Arakawa et al., Anal.Biochem. 333 (2004) 296-302, Guillory et al., Anal. Biochem. 39 (1971) 170-180, Cook et al., Anal. Biochem. 91 (1978) 557-565, Jansson et al., Anal. Biochem. 304 (2002) 135-137, Tagiri-Endo, Anal. Biochem. 315 (2003) 170-174, Drake et al., Anal. Biochem. 94 (1979) 117-120, Nyren et al., Anal. Biochem. 151 (1985) 504-509, und Barshop et al., Anal. Biochem. 197 (1991) 266-72, beschrieben.

Diese Verfahren sind mit dem Nachteil behaftet, daß die erforderlichen Enzyme und sonstigen Reagenzien häufig schwer zugänglich oder nur mit hohem Kostenaufwand zu beschaffen sind. Darüber hinaus sind diese Verfahren grundsätzlich störanfällig gegenüber Substanzen, die die Enzymaktivität der verwendeten Kopplungsenzyme modulieren; dies schränkt ihre Einsetzbarkeit insbesondere im Bereich der medizinischen Diagnostik und der biomedizinischen Forschung, beispielsweise dem pharmazeutischen Hochdurchsatz Screening, erheblich ein.

Im Stand der Technik ist darüber hinaus die Verwendung von Phosphat Nachweisen zur indirekten Detektion von Pyrophosphat bekannt; dabei wird Pyrophosphat zunächst enzymatisch oder chemisch gespalten und das entstehende Phosphat nachgewiesen und quantifiziert. Solche Verfahren werden beispielsweise in Fiske et al., J. Biol.Chem. 66 (1925) 375, Silcox et al., J. Clin. Invest. 52 (1973) 1863-1870, Gibson et al., Anal. Biochem. 254 (1997) 18-22, Cogan et al., Anal. Biochem. 271 (1999) 29-35, Upson et al., Anal. Biochem. 243 (1996) 41-45, WO 0042214 und Baykov et al., Anal. Biochem. 119 (1982) 211-213, dargelegt. Diese Verfahren sind typischerweise nicht für kontinuierliche Messungen geeignet und grundsätzlich störanfällig gegenüber Phosphat. Fabbrizzi et al., Angew. Chem. Int. Ed. 41 (2002) 3811-3814, beschreiben ein Nachweisverfahren mit Fluoreszenz Detektion, welches auf der Pyrophosphat vermittelten Verdrängung eines Indikatormoleküls von einem selektiven Rezeptormolekül beruht. Das Verfahren besitzt nur geringe Sensitivität mit Detektionsgrenze im mikromolaren Konzentrationsbereich.

Von ebenfalls geringer Sensitivität ist das Verfahren von Eriksson et al., Anal. Biochem. 293 (2001) 67-70, mit einer Detektionsgrenze im mittleren mikromolaren Konzentrationsbereich. Hierbei wird eine Luziferase-katalysierte Lumineszenzreaktion mit beiden Luziferin Enantiomeren, D- Luziferin und L-Luziferin, sowie einer geringen Menge ATP verwendet. Der Nachweis erfolgt über einen *hemmenden* Effekt von Pyrophosphat auf die Lumineszenzreaktion und die damit verbundene Abnahme der Lichtemission. Eriksson et al. beschreiben darüber hinaus die Verwendung dieses Nachweisverfahrens zur Bestimmung der Enzymaktivität von Pyrophosphatase; dabei wird vorgelegtes Pyrophosphat durch Pyrophosphatase abgebaut und der Anstieg der Lichtemission vermessen.

DE 19602662 beschreibt ein Verfahren zum Nachweis von Pyrophophat, bei dem ein vorinkubiertes Gemisch aus Luciferase, L-Luciferin, ATP und Pyrophosphatase mit der Pyrophosphat Testprobe versetzt wird und dabei einen Lichtblitz erzeugt; der Lichtblitz wird aufgezeichnet und für die Bestimmung der Pyrophosphat Konzentration herangezogen. Der Nachweis von Pyrophosphat in Konzentrationen von 10 µM bis 100 µM wird beschrieben. Das Verfahren ist nicht geeignet für kontinuierliche Messungen.

DE 10250491 beschreibt ein Nachweisverfahren für Pyrophosphat unter Verwendung einer Polymerase mit Pyrophosphorolyse-Aktivität und einem spezifisch markierten Oligonukleotid Indikatorsubstrat; zugesetztes Pyrophosphat führt zum pyrophosphorolytischen Abbau des Indikatorsubstrats unter Freisetzung eines markierten Mononukleotids, welches schließlich quantifiziert wird. Von Nachteil ist die Störanfälligkeit dieses Verfahrens gegenüber Nukleinsäure interkalierenden Substanzen, was die Verwendung im Bereich der pharmazeutischen Wirkstoffforschung, einschießlich dem pharmazeutischen Hochdurchsatz Screening, erheblich einschränkt.

WO 0036151 betrifft ein Verfahren zum Nachweis der enzymatischen Abspaltung von Pyrophosphat aus Nukleotidtriphosphaten. Die verwendeten Substrate sind Nukleotidtriphosphate, welche sowohl mit einem Fluorophor als auch mit einem Fluoreszenz Quencher modifiziert sind; die enzymatische Spaltung des Substrats setzt mit Fluorophor markiertes Pyrophosphat frei, welches nachgewiesen und schließlich quantifiziert wird. Aufgrund der eingeführten Markierungen sind die beschriebenen Substrate in Abhängigkeit des spaltenden Enzyms nur eingeschränkt anstelle der natürlichen Nukleotidtriphosphate einsetzbar.

Im Stand der Technik sind außerdem Verfahren zur Messung der Enzymaktivität von Guanylylcyclasen bekannt.

Guanlylylcyclasen (E.C. 4.6.1.2) katalysieren die Umsetzung von Guanosintriphosphat (GTP) in zyklisches 3'-5'-Guanosinmonophosphat (cGMP) und Pyrophosphat.

Domino et al., Methods in Enzymology 105 (1991) 345-355, beschreiben ein Verfahren unter Verwendung von radioaktiv markiertem GTP, welches im Verlauf der durch Guanlylylcyclase katalysierten Reaktion zu cGMP umgesetzt wird. Das gebildete cGMP wird von nicht umgesetztem GTP abgetrennt, durch Radioaktivitätsmessung quantifiziert und für die Berechnung der Enzymaktivität herangezogen. Das Verfahren umfasst mehrere aufwendige Arbeitschritte und lässt sich daher nur schwer automatisieren. Darüber hinaus ist das Verfahren nicht für die Durchführung kontinuierlicher Messungen geeignet.

Außerdem sind verschiedene Immunoassays unter Verwendung von cGMP spezifischen Antikörpern bekannt (Produktbeschreibung CatchPoint cGMP Fluorescent Assay Kit, Molecular Devices; Produktbeschreibung HTRF cGMP Assay, Cisbio international, Produktbeschreibung cGMP EIA Kit, Alexis Biochemicals, Produktbeschreibung HitHunter cGMP Assay, DiscoverRx). Dabei erfolgt der Nachweis des gebildeten cGMP indirekt über die Verdrängung und Quantifizierung einer an den Antikörper gebundenen Sonde (Kompetitiver Immunoassay). Nachteilig sind bei diesen Verfahren die hohen Reagenzienkosten oder präparativen Aufwendungen zur Bereitstellung der erforderlichen Antikörper, was die Verwendung in Hochdurchsatz Experimenten, wie beispielsweise dem pharmazeutischen Hochdurchsatz Screening, erheblich einschränkt. Die Verfahren sind zudem nicht für die Durchführung kontinuierlicher Messungen geeignet. Weiterhin umfassen mehrere Verfahren aufwendige Arbeitsschritte, insbesondere Waschprozeduren, welche eine effiziente Automatisierung erheblich erschweren.

US 20040229251 beschreibt ein Verfahren, bei dem Fluorophor markiertes GTP, speziell BODIPY markiertes GTP, als Surrogatsubstrat verwendet wird. Das beschriebene Verfahren ist mit dem intrinsischen Nachteil behaftet, daß die Reaktion nicht mit dem natürlichen Substrat ausgeführt wird, was zu systematischen Artefakten führen kann. Die hohen Reagenzienkosten oder präparativen Aufwendungen für die Bereitstellung des beschriebenen Surrogatsubstrats schränken zudem die Einsatzmöglichkeiten im Bereich von Hochdurchsatz Experimenten, einschließlich des pharmazeutischen Hochdurchsatz Screenings, ein.

Im Stand der Technik sind außerdem Verfahren zur Messung der Enzymaktivität von Squalensynthase bekannt.

Squalensynthase (E.C. 2.5.1.21) ist ein bifunktionelles Enzym, welches in einem ersten katalytischen Schritt die Kondensation von zwei Molekülen Farnesylpyrophosphat (FPP) zu Pre-squalenpyrophosphat vermittelt. In einem zweiten katalytischen Schritt wird Pre-squalenpyrophosphat zu Squalen umgesetzt. In beiden Schritten wird je ein Molekül Pryophosphat gebildet und zwei Moleküle NADPH verbraucht.

Es sind mehrere radioaktive Verfahren im Stand der Technik bekannt (A. Qureshi et al., J.Biol.Chem. 248 (1973) 1848, Ishihara et al., Bioorg.Med.Chem.11 (2003) 2403, H. Hiyoshi et al., J.Lip.Res. 41 (2000) 1136). Bei diesen Verfahren wird radioaktiv markiertes FPP als Substrat einsetzt und das während der Reaktion gebildete Squalen in organischen Lösungsmitteln aufgenommen, chromatographisch abgetrennt und schließlich quantifiziert. Diese Verfahren sind experimentell außerordentlich aufwendig und lassen sich nur schwer automatisieren. Darüber hinaus erlauben diese Verfahren keine kontinuierlichen Messungen.

US 20030157583 beschreibt ein Verfahren, das auf der Messung des NADPH Verbrauchs basiert, welcher, wie oben bereits dargelegt, mit dem Fortschreiten der Reaktion gekoppelt ist. Dieses Verfahren besitzt nur begrenzte Sensitivität und ist nicht für die Messung geringer Enzymaktivitäten geeignet.

Im Stand der Technik sind außerdem stabilisierte Lumineszenz-Systeme, die die Halbwertszeit der Lumineszenz-Reaktion unter Verwendung von Käfer-Luziferin/Luziferase verlängern (WO2006013837).

Weiterhin offenbart McElroy et al. (Arch. Biochem. Biophys. 46 (1953) 399-416) den Wirkungsweise von Pyrophosphat bei der.

Ahmad et al. beschreibt analytische Aspekte der Reaktionskinetik der Leuchtkäferlumineszenz-Reaktion (Ahmad et al. BIOLUMIN. CHEMILUMIN., [INT. SYMP. ANAL. APPL. BIOLUMIN. CHEMILUMIN.], 2ND, MEETING DATE 1980, 435-41. EDITOR(S): DELUCA, MARLENE A.; MCELROY, WILLIAM DAVID. PUBLISHER: ACADEMIC, NEW YORK, N. Y. CODEN: 45UJAC, 1981).

Weiterhin ist in Ronaghi M. et al. (Anal Biochem. 1996 Nov 1;242(1):84-9.) ein Verfahren zur Real-Time Sequenzierung mittels Bestimmung der Pyrophosphatfreisetzung offenbart.

DE19602662 beschreibt ein Verfahren zur bioluminometrischen Bestimmung unter Verwendung von L-Luziferin.

WO9640988 offenbart Quench-Reagenzien und Assays für enzymvermittelte Lumineszenz.

Zusätzlich wird im Stand der Technik der Einfluss von Dehydroluciferin, Co-Enzym A und Nikleosidtriphosphat auf die Lumineszenzreaktion beschrieben (Fontes et al, Biochem Biophys Res Commun. 1997 Aug 18;237(2):445-50).

Der Erfindung liegt nun die Aufgabe zu Grunde die aufgeführten Nachteile und Einschränkungen des Standes der Technik zu überwinden und ein Verfahren zum Nachweis von Pyrophosphat bereitzustellen, welches sich durch hohe Sensitivität und geringe Störanfälligkeit auszeichnet, sich leicht automatisieren und miniaturisieren lässt und sich zudem für die Durchführung kontinuierlicher Messungen eignet.

Die Aufgabe wird erfindungsgemäß durch Verfahren zum Nachweis von Pyrophosphat mit Biolumineszenz Detektion gelöst.

Gegenstand der Erfindung sind Verfahren zum Nachweis von Pyrophosphat, welche folgende Stufen umfassen:
(a) Bereitstellen einer Zusammensetzung umfassend Dehydro-Luziferin, Luziferin, ATP und einer Luziferase, die durch Pyrophosphat aktivierbar ist, z.B. einer Luziferase aus *Photinus pyralis;*
(b) In Kontakt bringen der Zusammensetzung mit der Testprobe; und
(c) Messung von Lumineszenz.

Die in Stufe (a) des erfindungsgemäßen Verfahrens verwendete Zusammensetzung ist in einer bevorzugten Ausgestaltung eine wässrige Lösung umfassend Dehydro-Luziferin, Luziferin, ATP und *Photinus pyralis* Luziferase und kann durch Kombination von wässrigen Lösungen der einzelnen Komponenten erhalten werden. Luziferin, ATP und *Photinus pyralis* Luziferase sind kommerziell erhältlich, wobei Produkte von hoher Reinheit und im Fall von *Photinus pyralis* Luziferase zudem von hoher spezifischer Enzymaktivität bevorzugt sind. Dehydro-Luziferin ist nach einem im Stand der Technik beschriebenen Verfahren aus kommerziell erhältlichem Luziferin leicht zugänglich (Bitler & McElroy, Arch. Biochem. Biophys. 72 (1957) 358). Wässrige Lösungen der einzelnen Komponenten lassen sich problemlos in den erforderlichen Konzentrationen darstellen.

Gegenstand der Erfindung ist die Verwendung von nicht-rekombinanten oder rekombinanten Luziferasen und daraus abgeleiteten oder mutierten Varianten, dadurch gekennzeichnet, dass sie bioluminogene biochemische Reaktionen katalysieren.

Gegenstand der Erfindung ist die Verwendung von nicht-rekombinanten oder rekombinanten Luziferasen, deren bioluminogene biochemische Reaktion durch Pyrophosphat aktivierbar ist, und daraus abgeleitete oder mutierte Varianten. Eine Aktivierung einer bioluminogenen biochemischen Reaktion einer Luziferase kann durch Aufhebung einer Produkthemmung oder durch direkte, zum Beispiel allosterische, Enzymaktivierung oder durch andere Mechanismen stattfinden.

Gegenstand der Erfindung ist die Verwendung von Luziferasen und rekombinanten Luziferasen, ursprünglich isoliert aus Insekten der Superfamilien Elateroidea und Cantharoidea, dadurch gekennzeichnet, dass sie ATP und Luziferin konsumierende bioluminogene biochemische Reaktionen katalysieren, zum Beispiel ausgewählt, aber nicht eingeschränkt durch, aus den Insekten *Photinus pyralis, Pyrophorus plagiophthalamus, Luciola cruciata, Luciola lateralis* oder *Luciola mingrelica* (US 6.387.675 B1, Wood et al., J. Biolum. Chemilum. 4 (1989) 289-301, Wood et al., J. Biolum. Chemilum. 4 (1989) 31-39, Tatsumi et al., Biochim. Biophys. Acta 1131 (1992) 161-165). Ein besonders bevorzugter Gegenstand der Erfindung ist die Verwendung der Luziferase aus Photinus pyralis.

Gegenstand der Erfindung ist die Verwendung von Luziferase-Mutanten, die sich dadurch auszeichnen, dass sie veränderte spektrale Biolumineszenz-Eigenschaften besitzen, zum Beispiel ausgewählt, aber nicht eingeschränkt, durch Substitutionen die zu den Aminosäurepositionen 215, 224, 232, 236, 237, 242, 244, 245, 248, 282, 283 oder 348 der Luziferase LucPplGR aus *Pyrophorus plagiophthalamus* korrespondieren (US 6.387.675 B1).

Ein weiterer Gegenstand der Erfindung ist die Verwendung von Luziferase-Mutanten, die sich dadurch auszeichnen, dass sie veränderte thermische Stabilität besitzen, zum Beispiel ausgewählt, aber nicht eingeschränkt, durch Substitutionen der zu Aminosäureposition 217 der Luziferasen aus *Luciola cruciata oder Luciola lateralis* korrespondiert durch eine hydrophobe Aminosäure (US 5.229.285).

Ein weiterer Gegenstand der Erfindung ist die Verwendung von Luziferase-Mutanten, die sich dadurch auszeichnen, dass sie veränderte thermische Stabilität besitzen, zum Beispiel ausgewählt, aber nicht eingeschränkt, durch Substitutionen der zu Aminosäureposition 286 (Serin durch Asparagin), Aminosäureposition 326 (Glycin durch Serin), Aminosäureposition 433 (Histidin durch Tyrosin) oder Aminosäureposition 452 (Prolin durch Serin) der Luziferase aus *Luciola cruciata* korrespondiert (US 5.219.737).

Die bevorzugte Ausgestaltung der Zusammensetzung als wässrige Lösung umfassend die aufgelisteten Komponenten enhält
Dehydro-Luziferin in einem bevorzugten Konzentrationsbereich von 5 µM bis 250 µM, wobei der Bereich von 10 µM bis 100 µM mehr bevorzugt und der Bereich von 20 µM bis 40 µM meisten bevorzugt ist,
Luziferin in einem bevorzugten Konzentrationsbereich von 10 µM bis 500 µM, wobei der Bereich von 30 µM bis 300 µM mehr bevorzugt und der Bereich von 70 µM bis 200 µM am meisten bevorzugt ist,
ATP in einem bevorzugten Konzentrationsbereich von 10 µM bis 500 µM, wobei der Bereich von 30 µM bis 300 µM mehr bevorzugt und der Bereich von 70 µM bis 200 µM am meisten bevorzugt ist, und
*Photinus pyralis* Luziferase in einem bevorzugten Konzentrationsbereich von 0,1 nM bis 10 nM, wobei der Bereich von 0,3 nM bis 3 nM mehr bevorzugt und der Bereich von 0,5 nM bis 2 nM am meisten bevorzugt ist.

Die in Stufe (a) des erfindungsgemäßen Verfahrens verwendete Zusammensetzung kann zusätzliche Komponenten beispielsweise Puffersubstanzen, monovalente und divalente Salze, Reduktionsmittel, insbesondere solche, die freie Mercapto-Gruppen enthalten, Proteine, synthetische Polymere und Detergenzien enthalten.

Beispiele für Puffersubstanzen sind HEPES, TEA, Tris, Tricine, MES und MOPS, wobei TEA und Tris bevorzugt sind. Puffersubstanzen sind vorzugsweise in Konzentrationen von 10 mM bis 100 mM enthalten. Der pH Wert liegt vorzugsweise im Bereich von 7,0 bis 8,0, noch mehr bevorzugt im Bereich von 7,3 bis 7,7.

Beispiele für monovalente Salze sind NaCl, KCl, NH4Cl, Na-Acetat, K-Acetat, NH4-Acetat, wobei NaCl und KCl bevorzugt ist. Monovalente Salze sind vorzugsweise in Konzentrationen von 0 mM bis 200 mM enthalten.

Beispiele für divalente Salze sind MgCl2, MgSO4, Mg-Acetate, MnCL2 und CaCl2, wobei MgCl2 bevorzugt ist. Divalente Salze sind vorzugsweise in Konzentrationen von 1 mM bis 10 mM enthalten.

Beispiele für Reduktionsmittel, insbesondere solche, die freie Mercapto-Gruppen enthalten, sind Mercapto-ethanol, DTE, DTT und Glutathion, wobei DTT bevorzugt ist. Reduktionsmittel sind vorzugsweise in Konzentrationen von 0,2 mM bis 20 mM enthalten.

Beispiele für zusätzliche Proteine oder synthetische Polymere sind BSA, HSA, Hämoglobin, Casein und PEG, wobei BSA bevorzugt ist. Zusätzliche Proteine oder synthetische Polymere sind vorzugsweise in Konzentrationen von 0,01% bis 1% (Gewicht pro Volumen) enthalten.

Beispiele für Detergenzien sind Brij, Tween, CHAPS, TRITON und NP-40, wobei Brij und CHAPS bevorzugt sind. Detergenzien sind vorzugsweise in Konzentrationen von 0,001% bis 0,01% (Gewicht pro Volumen) enthalten.

Eine besonders bevorzugte Ausgestaltung der Zusammensetzung enthält als zusätzliche Komponenten 50 mM TEA (pH = 7,5), 2 mM MgCl2, 0,4 mM DTT, 0,1% (w/v) BSA (Fraktion V), und 0,005% (w/v) Brij-35.

Wie oben bereits zum Ausdruck gebracht, kann die bevorzugte Ausgestaltung der Zusammensetzung als wässrige Lösung umfassend Dehydro-Luziferin, Luziferin, ATP und *Photinus pyralis* Luziferase durch Kombination von wässrigen Lösungen der einzelnen Komponenten erhalten werden; dabei werden vorzugsweise zunächst wässrige Lösungen von *Photinus pyralis* Luziferase, Dehydro-Luziferin und ATP kombiniert und abschließend Luziferin, vorzugsweise in wässriger Lösung, zugesetzt. Die zunächst kombinierten Lösungen von *Photinus pyralis* Luziferase, Dehydro-Luziferin und ATP können vor dem Zusatz des Luziferins inkubiert werden, wobei eine Inkubationszeit im Bereich von 1 min bis 15 min und eine Inkubationstemperatur im Bereich von 15°C bis 30°C bevorzugt sind.

Besonders bevorzugt ist die Herstellung einer in Stufe (a) des erfindungsgemäßen Verfahrens bevorzugt verwendeten Zusammensetzung nach folgendem Verfahren umfassend die Stufen:
(a) Kombination wässriger Lösungen von *Photinus pyralis* Luziferase, Dehydro-Luziferin und ATP, welche jeweils als zusätzliche Komponenten 50 mM TEA (pH = 7,5), 2 mM MgCl₂, 0,4 mM DTT, 0,1% (w/v) BSA (Fraktion V), und 0,005% (w/v) Brij-35 enthalten,
(b) Inkubation der Kombination aus (a) über 5 min bis 10 min bei einer Temperatur von 20°C bis 25 °C;
(c) Ergänzen der inkubierten Kombination aus (b) mit einer wässrigen Lösung von Luziferin, welche als zusätzliche Komponenten 50 mM TEA (pH = 7,5), 2 mM MgCl₂, 0,4 mM DTT, 0,1% (w/v) BSA (Fraktion V), und 0,005% (w/v) Brij-35 enthält.

Eine in Stufe (a) des erfindungsgemäßen Verfahrens verwendete Zusammensetzung umfassend in wässriger Lösung Dehydro-Luziferin, Luziferin, ATP und *Photinus pyralis* Luziferase sowie zusätzliche Komponenten kann gelagert werden. Die Lagerstabilität der Zusammensetzung hängt unter anderem von der Auswahl und Konzentration der zusätzlichen Komponenten und von den Lagerungsbedingungen ab. Typischerweise wird eine Lagerstabilität von mehreren Tagen bis Wochen erreicht. Bevorzugte Lagerungstemperaturen liegen im Bereich von 2°C bis 10°C. Die Zusammensetzung kann auch in gefrorenem Zustand, vorzugsweise unter minus 70°C, gelagert werden; dabei ist das Einfrieren der Zusammensetzung möglichst schnell, vorzugsweise schockartig, durchzuführen.

Eine in Stufe (a) des erfindungsgemäßen Verfahrens verwendete Zusammensetzung umfassend in wässriger Lösung Dehydro-Luziferin, Luziferin, ATP und *Photinus pyralis* Luziferase sowie als zusätzliche Komponenten 50 mM TEA (pH = 7,5), 2 mM MgCl₂, 0,4 mM DTT, 0,1% (w/v) BSA (Fraktion V), und 0,005% (w/v) Brij-35 kann typischerweise über mehrere Tage, mindestens jedoch 24 Stunden, bei 4°C bis 10°C gelagert werden.

Die Testprobe ist vorzugsweise eine im wesentlichen wässrige Probe, wobei es sich um eine als solche bereits im wesentlichen wässrige Probe oder das Produkt einer zuvor durchgeführten Probenaufarbeitung, beispielsweise einer Extraktion, handeln kann.

Die Testprobe kann aus einer natürlichen Quelle umfassend die belebte und unbelebte Umwelt stammen. Beispiele für Testproben aus der unbelebten Umwelt sind Wasserproben oder Extrakte aus Erde, Sand oder Gestein. Beispiele für Testproben aus der belebten Umwelt sind Proben aus tierischen oder pflanzlichen Organismen. Von besonderem Interesse sind Proben aus dem menschlichen Körper, wobei es sich um Gewebeproben oder Proben von Körperflüssigkeiten oder Aufarbeitungen daraus handeln kann. Beispiele für solche Körperflüssigkeiten sind Blut, Serum, Urin, Rückenmarksflüssigkeit, Gelenkflüssigkeit oder Aufarbeitungen daraus.

Die Testprobe kann darüber hinaus auch eine chemische Reaktion sein, bei der Pyrophosphat entsteht oder verbraucht wird. Von besonderem Interesse sind dabei enzymkatalysierte chemische Reaktionen (Enzymreaktionen), bei denen Pyrophosphat entsteht oder verbraucht wird. Solche Reaktionen werden zum Beispiel von Guanylylcyclasen, Adenylylcyclasen, DNA- oder RNA-Polymerasen, Squalensynthase oder Pyrophosphatase katalysiert.

In einer bevorzugten Ausgestaltung wird das erfindungsgemäße Verfahren in homogener Phase durchgeführt. In dieser Ausgestaltung lässt sich das erfindungsgemäße Verfahren besonders leicht automatisieren und miniaturisieren. Dafür wird in Stufe (b) des Verfahrens die Zusammensetzung als wässrige Lösung umfassend Dehydro-Luziferin, Luziferin, ATP und *Photinus pyralis* Luziferase mit der im wesentlichen wässrigen Testprobe kombiniert. Das resultierende Testvolumen liegt vorzugsweise unter 1000 µL, mehr bevorzugt unter 100 µL, am meisten bevorzugt unter 10 µL.

Die Kombination von Zusammensetzung und Testprobe kann mit handelsüblichen Vorrichtungen zur Handhabung von Flüssigkeiten (Liquid Handling Systems) durchgeführt werden. Beispiele für geeignete Vorrichtungen sind Pipettiervorrichtungen, einschließlich Mikropipetten und Parallelpipettierer, Dispensiervorrichtungen, einschließlich Piezo-Effekt basierender Systeme, Bubble-Jet Systeme sowie Dispensiergeräte mit Ventilsteuerung, und Übertragungsvorrichtungen, einschließlich Pin-Tool basierender Systeme.

Das erfindungsgemäße Verfahren kann in handelsüblichen Reaktionsgefäßen, vorzugsweise Mikroplatten mit 96, 384 oder 1536 Vertiefungen, ausgeführt werden. Mikroplatten mit 1536 Vertiefungen sind besonders bevorzugt. Als Reaktionsräume eignen sich außerdem Kapillaren oder Kapillarsysteme sowie Hohlräume poröser Materialien, einschließlich Polymergele und poröser Mikrokügelchen (Beads). Darüber hinaus ist eine Durchführung auf Mikröträgern (Chips) oder in Flüssigkeitsfilmen auf mikroskopischen Objektträgern oder auf Mikrokügelchen (Beads) denkbar. Das Verfahren könnte auch als Prozess oder Teilprozess eines Lab-on-the-Chip Konzepts ausgeführt werden.

In einer weiteren Ausgestaltung kann das erfindungsgemäße Verfahren auch in einem heterogenen Phasengefüge durchgeführt werden. Dabei liegt mindestens eine Komponente der Testprobe oder der Zusammensetzung in einer weiteren Phase vor.

In einer bevorzugten Ausgestaltung wird *Photinus pyralis* Luziferase auf einem geeigneten festen Träger immobilisiert und durch In-Kontakt-bringen mit einer wässrigen Zusammensetzung umfassend Dehydro-Luziferin, Luziferin und ATP aktiviert. Anschließend wird die im wesentlichen wässrige Testprobe mit der immobilisierten Luziferase in Kontakt gebracht, vorzugsweise im Gemisch mit der aktivierenden Zusammensetzung, und die emittierte Lumineszenz gemessen. Die Immobilisierung der Luziferase kann, je nach Vorrichtung, zum Beispiel auf der Oberfläche von Kapillaren, Kapillarsystemen, Mikroträgern (Chips) oder Mikrokügelchen (Beads) erfolgen.

Die in Stufe (c) des erfindungsgemäßen Verfahrens zu messende Lumineszenz korreliert mit der Pyrophosphatkonzentration in der Testprobe (vergleiche Beispiel 1), wobei eine höhere Pyrophosphatkonzentration zu einem stärkeren Lumineszenzsignal führt. Im Konzentrationsbereich unter 20 µM Pyrophosphat wird typischerweise eine lineare Korrelation zwischen Lumineszenzsignal und Pyrophosphatkonzentration beobachtet (vergleiche Beispiel 1). Das erfindungsgemäße Verfahren ist daher besonders geeignet für den Nachweis von Pyrophosphat in Konzentrationen unter 20 µM. Der bevorzugte Bereich ist eine Pyrophosphatkonzentration in der Testprobe von 20 nM bis 20 µM, mehr bevorzugt ist der Bereich von 50 nM bis 10 µM, am meisten bevorzugt ist der Bereich von 100 nM bis 1 µM.

Für eine quantitative Bestimmung der Pyrophosphatkonzentration in der Testprobe werden zusätzliche Referenzproben mit bekannter Pyrophosphatkonzentration in gleicher Weise behandelt und vermessen wie die Testprobe; aus dieser zusätzlichen Untersuchung wird eine Korrelation von Lumineszenzsignal und Pyrophosphatkonzentration unter den gegebenen experimentellen Bedingungen abgeleitet, wobei die Messdaten zum Beispiel graphisch interpoliert oder durch elektronische Prozessierung angenähert werden können. Aus der abgeleiteten Korrelation lässt sich dem für die Testprobe gemessenen Lumineszenzwert direkt eine Pyrophosphatkonzentration zuordnen.

Die Messung von Lumineszenz in Stufe (c) des erfindungsgemäßen Verfahrens kann mit handelsüblichen Lumineszenzmessgeräten durchgeführt werden; es können jedoch je nach Ausgestaltung des Verfahrens, insbesondere des Reaktionsgefäßes, spezielle Lesegeräte erforderlich sein.

Der Testansatz wird vorzugsweise direkt im Reaktionsgefäß vermessen; dabei sind Messungen direkt in Mikroplatten mit 96, 384 oder 1536 Vertiefungen bevorzugt. Je nach Ausgestaltung des Verfahrens kann es aber auch vorteilhaft sein, den Testansatz ganz oder teilweise in ein weiteres für die Messung geeignetes Gefäß zu überführen und erst dann zu vermessen.

Soll die Pyrophosphatkonzentration in der Testprobe zu verschiedenen Zeitpunkten bestimmt werden, kann die Messung der Lumineszenz in Stufe (c) des erfindungsgemäßen Verfahrens wiederholt durchgeführt werden, wobei der Testansatz direkt im Reaktiongefäß vermessen wird, oder zu den gewünschten Zeitpunkten Aliquots in weitere für die Messung geeignete Gefäße überführt und vermessen werden. In beiden Fällen sollte die Detektionsreaktion möglichst nicht mit denjenigen Prozessen in der Testprobe interferieren, welche die Pyrophosphatkonzentration bestimmen oder beeinflussen. In einem alternativen Vorgehen kann man zu gewünschten Zeitpunkten auch direkt aus der Testprobe Aliquots entnehmen, diese Aliquots mit der Zusammensetzung in Kontakt bringen und dann vermessen.

Nachdem die Testprobe in Stufe (b) des erfindungsgemäßen Verfahrens mit der Zusammensetzung in Kontakt gebracht worden ist, bildet sich das resultierende und in Stufe (c) zu vermessende Lumineszenzsignal schnell, typischerweise innerhalb von Sekunden, aus. Das Signal besitzt in der Regel eine Intensität, die mit handelsüblichen Lumineszenzmessgeräten in kurzen Intergrationszeiten, typischerweise innerhalb von Sekunden, zuverlässig bestimmt werden kann.

Das erfindungsgemäße Verfahren ermöglicht daher eine besonders schnelle, zeitnahe Bestimmung von Pyrophosphatkonzentrationen. Aufgrund der Schnelligkeit eignet sich das Verfahren bevorzugt auch für eine zeitlich aufgelöste Bestimmung der Pyrophosphatkonzentration in einer Testprobe, wobei in der oben bereits ausgeführten Weise vorgegangen werden kann.

Zeitlich aufgelöste Bestimmungen von Pyrophosphatkonzentrationen sind insbesondere von Interesse, wenn es sich bei der Testprobe um chemische, einschließlich enzymkatalysierte chemische Reaktionen handelt, bei denen Pyrophosphat gebildet oder verbraucht wird. Auf Grundlage des zeitlichen Verlaufs der Pyrophosphatkonzentration kann die Umsatzgeschwindigkeit der untersuchten Reaktion und auch die Aktivität des gegebenenfalls eingesetzten Enzyms auf bekannte Weise berechnet werden.

Ein weiterer Gegenstand der Erfindung sind Verfahren zum Nachweis von Pyrophosphat, welche folgende Stufen umfassen:
(a) In Kontakt bringen der Komponenten einer Zusammensetzung umfassend Dehydro-Luziferin, Luziferin, ATP und und einer Luziferase, die durch Pyrophosphat aktivierbar ist, z.B. einer Luziferase aus *Photinus pyralis;* mit der Testprobe
(b) Messung von Lumineszenz.

Die Stufe (a) des Verfahrens sollte je nach Reihenfolge, in der die Komponenten der Zusammensetzung und die Testprobe kombiniert werden, vorzugsweise zügig, das heißt innerhalb von Minuten, durchgeführt werden. Das in Stufe (b) des Verfahrens zu vermessende Lumineszenzsignal könnte sich je nach Reihenfolge, in der die Komponenten der Zusammensetzung und die Testprobe kombiniert werden, und in Abhängigkeit zusätzlicher Komponenten zunächst noch über ein kurzes Zeitintervall entwickeln und erst dann stabilisieren; für die Bestimmung der Pyrophosphatkonzentration ist in diesen Fällen die stabilisierte Lumineszenzintensität heranzuziehen.

Die Zusammensetzung sowie die umfassten einzelnen Komponenten werden vorzugsweise als wässrige Lösungen ausgestaltet und kombiniert; die Lösungen können, wie oben bereits dargelegt, zusätzliche Komponenten enthalten.

Das Kombinieren der wässrigen Komponenten sowie das In-Kontakt-bringen mit der Testprobe als auch die anschließende Messung der Lumineszenz kann in der bereits oben erläuterten Weise durchgeführt werden.

In einer bevorzugten Ausgestaltung des Verfahrens wird in Stufe (a) zunächst eine Kombination umfassend Dehydro-Luziferin, ATP und *Photinus pyralis* Luziferase bereitgestellt; die Kombination wird dann mit der Testprobe in Kontakt gebracht und schließlich Luziferin addiert.

Die Kombination umfassend Dehydro-Luziferin, ATP und *Photinus pyralis* Luziferase kann gelagert werden. Die Lagerstabilität der Kombination hängt unter anderem von der Auswahl und Konzentration der zusätzlichen Komponenten und von den Lagerungsbedingungen ab.

Gegenstand der Erfindung sind außerdem Verfahren zur Messung des zeitlichen Verlaufs einer Pyrophosphat bildenden oder verbrauchenden Enzymreaktion, welche folgende Stufen umfassen:
(a) Bereitstellen einer Zusammensetzung umfassend Dehydro-Luziferin, Luziferin, ATP und und einer Luziferase, die durch Pyrophosphat aktivierbar ist, z.B. einer Luziferase aus *Photinus pyralis;*
(b) In Kontakt bringen der Zusammensetzung mit der Pyrophosphat bildenden oder verbrauchenden Enzymreaktion; und
(c) Kontinuierliche Messung von Lumineszenz.

Die Zusammensetzung umfassend Dehydro-Luziferin, Luziferin, ATP und *Photinus pyralis* Luziferase wird, wie oben bereits dargelegt, vorzugsweise als wässrige Lösung ausgestaltet und kann zusätzliche Komponenten enthalten.

Das Bereitstellen der Zusammensetzung durch Kombinieren der vorzugsweise wässrigen Komponenten sowie das In-Kontakt-bringen mit der Testprobe können ebenfalls in der weiter oben erläuterten Weise durchgeführt werden.

Für die kontinuierliche Messung von Lumineszenz in Stufe (c) des Verfahrens werden in kurzem Zeitabstand Einzelmessungen mit kurzen Integrationsintervallen durchgeführt. Mit handelsüblichen Geräten können typische Lumineszenzsignale mit Integrationsintervallen im Bereich von wenigen Sekunden, zum Teil auch von weniger als einer Sekunde, zuverlässig und in kurzem Zeitabstand, das heißt in einem Zeitabstand von weniger als einer Sekunde, vermessen werden.

Das erfindungsgemäße Verfahren ermöglicht, wie oben bereits zum Ausdruck gebracht, eine besonders schnelle, zeitnahe Bestimmung der jeweiligen Pyrophosphatkonzentration. Mit Hilfe der beschriebenen kontinuierlichen Lumineszenzinessung kann der Verlauf der Pyrophosphatkonzentration mit hoher zeitlicher Auflösung bestimmt und verfolgt werden. Bei einer Pyrophosphat bildenden oder verbrauchenden Reaktion kann der Zeitverlauf der Pyrophosphatkonzentration als Indikator für den Verlauf der Reaktion herangezogen und der jeweils erreichte Reaktionsumsatz sowie die jeweilige Reaktionsgeschwindigkeit, einschließlich der jeweiligen Aktivität des eingesetzten Enzyms, auf bekannte Weise berechnet werden.

Der Beginn und die Dauer der kontinuierlichen Lumineszenzmessung in Stufe (c) des Verfahrens kann je nach experimenteller Zielsetzung variiert werden. In einer bevorzugten Ausgestaltung wird die Enzymreaktion unmittelbar vor Beginn der Lumineszenzmessung initiiert. In dieser Weise kann die in vielen enzymkinetischen Untersuchungen angestrebte Bestimmung von Anfangsreaktionsgeschwindigkeiten erreicht werden.

Gegenstand der Erfindung sind außerdem Verfahren zur Messung des zeitlichen Verlaufs einer Pyrophosphat bildenden oder verbrauchenden Enzymreaktion, welche folgende Stufen umfassen:
(a) In Kontakt bringen einer oder mehrerer Komponenten der Pyrophosphat bildenden oder verbrauchenden Enzymreaktion mit einer Zusammensetzung umfassend Dehydro-Luziferin, Luziferin, ATP und und einer Luziferase, die durch Pyrophosphat aktivierbar ist, z.B. einer Luziferase aus *Photinus pyralis;*
(b) In Kontakt bringen der Kombination aus (a) mit den restlichen Komponenten der Pyrophosphat bildenden oder verbrauchenden Enzymreaktion; und
(c) Kontinuierliche Messung von Lumineszenz.

Die Zusammensetzung umfassend Dehydro-Luziferin, Luziferin, ATP und *Photinus pyralis* Luziferase wird, wie oben bereits dargelegt, vorzugsweise als wässrige Lösung ausgestaltet und kann zusätzliche Komponenten enthalten.

Das Bereitstellen der Zusammensetzung durch Kombinieren der vorzugsweise wässrigen Komponenten, das In-Kontakt-bringen mit den Komponenten der Pyrophosphat bildenden oder verbrauchenden Enzymreaktion und die kontinuierliche Messung von Lumineszenz können ebenfalls in der weiter oben erläuterten Weise durchgeführt werden.

In einer bevorzugten Ausgestaltung wird die Kombination aus Stufe (a) des Verfahrens vor Beginn von Stufe (b) inkubiert; die Inkubation erfolgt vorzugsweise bei der vorgesehenen Reaktionstemperatur, zum Beispiel 37°C, und über einen Zeitraum, der ausreicht, um wenigstens das gesamte Volumen der Kombination auf die vorgesehene Reaktionstemperatur zu bringen. Bevorzugt ist darüber hinaus, die Stufe (a) derart auszugestalten, dass in Stufe (b) nur noch eine restliche Komponente der Pyrophosphat bildenden oder verbrauchenden Enzymreaktion mit der Kombination aus Stufe (a) in Kontakt gebracht wird und dadurch die Reaktion initiiert wird (vergleiche Beispiel 2 : Messung einer durch lösliche vaskuläre Guanylatcyclase katalysierten Enyzmreaktion). Dabei wird die eine restliche Komponente vorzugsweise mit Hilfe einer Dispensiervorrichtung direkt im Lumineszenzmessgerät zugesetzt, so dass die kontinuierliche Messung von Lumineszenz unmittelbar, das heißt mit einem zeitlichen Abstand von typischerweise weniger als einer Sekunde, gestartet werden kann. Alternativ kann die Messung auch bereits vor Zusatz der einen restlichen Komponente gestartet werden, wobei für die Bestimmung der Anfangsreaktionsgeschwindigkeit oder der Enzymaktivität die entsprechenden Abschnitte der Messung herangezogen werden.

Gegenstand der Erfindung sind darüber hinaus Verfahren zur Messung des zeitlichen Verlaufs einer Pyrophosphat bildenden oder verbrauchenden Enzymreaktion, welche folgende Stufen umfassen:
(a) In Kontakt bringen der Komponenten der Pyrophosphat bildenden oder verbrauchenden Enzymreaktion mit den Komponenten einer Zusammensetzung umfassend Dehydro-Luziferin, Luziferin, ATP und einer Luziferase, die durch Pyrophosphat aktivierbar ist, z.B. einer Luziferase aus *Photinus pyralis;*
(b) Kontinuierliche Messung von Lumineszenz.

Die Zusammensetzung umfassend Dehydro-Luziferin, Luziferin, ATP und *Photinus pyralis* Luziferase wird, wie oben bereits dargelegt, vorzugsweise als wässrige Lösung ausgestaltet und kann zusätzliche Komponenten enthalten. Das In-Kontakt-bringen der Komponenten der Zusammensetzung mit den Komponenten der Pyrophosphat bildenden oder verbrauchenden Enzymreaktion und die kontinuierliche Messung von Lumineszenz können ebenfalls in der weiter oben erläuterten Weise durchgeführt werden.

Die Stufe (a) des Verfahrens sollte je nach Reihenfolge, in der die Komponenten der Zusammensetzung und die Komponenten der Pyrophosphat bildenden oder verbrauchenden Enzymreaktion kombiniert werden, vorzugsweise zügig, das heißt innerhalb von Minuten, durchgeführt werden. Das in Stufe (b) des Verfahrens zu vermessende Lumineszenzsignal könnte sich je nach Reihenfolge, in der die Komponenten der Zusammensetzung und die Komponenten der Pyrophosphat bildenden oder verbrauchenden Enzymreaktion kombiniert werden, und in Abhängigkeit zusätzlicher Komponenten zunächst noch über ein kurzes Zeitintervall entwickeln und erst dann stabilisieren; für die Bestimmung der Reaktionsgeschwindigkeit oder der Enzymaktivität sind in diesen Fällen geeignete spätere Abschnitte der kontinuierlichen Lumineszenzmessung heranzuziehen.

Die erfindungsgemäßen Verfahren können in verschiedenen Bereichen vorteilhaft eingesetzt werden; von besonderem Interesse ist der Einsatz im Bereich der medizinischen Diagnostik, der biomedizinischen Forschung, insbesondere der Wirkstoffforschung und der Arzneimittelforschung, sowie im Bereich der Umwelt- und Lebensmittelanalytik. Dabei kann sich der Einsatz der erfindungsgemäßen Verfahren neben der Bestimmung von Pyrophosphatkonzentrationen auch auf die Messung von chemischen Reaktionsumsätzen und Enzymaktivitäten richten.

Die erfindungsgemäßen Verfahren können, wie oben bereits erläutert, in homogener Phase und in wenigen einfachen Prozessierungsstufen durchgeführt werden; dadurch eignen sie sich ausgezeichnet zur Automatisierung und Miniaturisierung.

Im Bereich der medizinischen Diagnostik bietet sich der Einsatz der erfindungsgemäßen Verfahren überall dort an, wo der Pyrophosphatgehalt der Testprobe, ein Indikator für eine krankhafte Veränderung sein kann. Besonders interessant können Proben von Körperflüssigkeiten oder Aufarbeitungen von Gewebe sein, zum Beispiel Proben von Blut, Serum, Urin, Rückenmarksflüssigkeit, Gelenkflüssigkeit oder Aufarbeitungen daraus. Die erfindungsgemäßen Verfahren eignen sich aufgrund der hohen Sensitivität und geringen Störanfälligkeit, insbesondere gegenüber typischen Probenbestandteilen, wie Phosphat oder ATP, ausgezeichnet zur Analyse derartiger Testproben. Darüber hinaus ermöglicht die leichte Automatisierbarkeit und Miniaturisierbarkeit der erfindungsgemäßen Verfahren eine besonders effiziente und kostengünstige Durchführung der Analysen.

Der automatisierte Einsatz der erfindungsgemäßen Verfahren kann darüber hinaus im Bereich der bio-medizinischen Forschung, insbesondere der pharmazeutischen Wirkstoffforschung, von Bedeutung sein; dort werden zur Auffindung neuer Wirkstoffkandidaten regelmäßig große Substanzbibliotheken mit teilweise mehr als einer Million Substanzen mit Hilfe automatisierter Verfahren durchgemustert (Hochdurchsatz Screening; High Throughput Screening); neben der leichten Automatisierbarkeit der Verfahren ist bei dieser Anwendung vor allem die Miniaturisierbarkeit, die hohe Sensitivität und die geringe Störanfälligkeit von Vorteil. Die Miniaturisierung der Testformate und die hohe Sensitivität reduzieren die Aufwendungen für die Herstellung oder Beschaffung von Reagenzien sowie den Verbrauch von Testsubstanzen. Geringe Störanfälligkeit führt üblicherweise zu hoher Datenqualität und guter Reproduzierbarkeit der Testergebnisse.

Das pharmazeutische Hochdurchsatz Screening richtet sich typischerweise auf die Identifizierung von Modulatoren einer biologischen Aktivität, beispielsweise einer Enyzmaktivität, welche das Ziel einer medizinischen Therapie sein kann. Die erfindungsgemäßen Verfahren eignen sich ausgezeichnet für einen solchen Einsatz im pharmazeutischen Hochdurchsatz Screening, speziell zur Auffindung von Modulatoren von Enzymen, die Pyrophosphat bildende oder verbrauchende Reaktionen katalysieren; von besonderem Interesse sind dabei zum Beispiel Modulatoren der Enzymaktivität von Guanylylcyclasen, Adenylylcyclasen, DNA- oder RNA- Polymerasen oder von Squalensynthase. Dafür wird die Aktivität des betreffenden Enzyms nach einem der oben beschriebenen erfindungsgemäßen Verfahren in Gegenwart einer oder mehrerer geeigneter Konzentrationen der Testsubstanz bestimmt, mit der Aktivität des Enzyms in Abwesenheit der Testsubstanz verglichen und daraus die modulierenden Eigenschaften der Testsubstanz in bekannter Weise abgeleitet. Üblicherweise abgeleitete Eigenschaften einer Testsubstanz sind die aktivierende oder inhibierende Wirkung auf die untersuchte Enzymaktivität (vergleiche Beispiele 3 und 5).

Darüber hinaus können die erfindungsgemäßen Verfahren auch zur weiteren enzymkinetischen Charakterisierung von Modulatoren verwendet werden, beispielsweise zur Bestimmung des Inhibitionsmechanismus, der Bildungs- und Dissoziationsgeschwindigkeit des Enyzm-Modulator-Komplexes oder der mit der Bildung und Dissoziation des Enyzm-Modulator-Komplexes verbundenen Energieumsätze. Außerdem können die Verfahren erfindungsgemäß bei der Charakterisierung von Enzymen und Enzymreaktionen, bei denen Pyrophosphat gebildet oder verbraucht wird, einschließlich der Charakterisierung von Substrateigenschaften sowie der Auffindung und Optimierung von Surrogatsubstraten, vorteilhaft eingesetzt werden.

Die Vorteile der erfindungsgemäßen Verfahren, insbesondere die geringe Störanfälligkeit gegenüber Phosphat und ATP, können außerdem bei Anwendungen im Bereich der Genomanalyse und der Genexpressionsanalyse genutzt werden. Diesen Anwendungen liegt der Nachweis und gegebenenfalls die Bestimmung der enzymatischen Aktivität von DNA- oder RNA-abhängigen Polymerasen, einschließlich Reverser Transkriptasen, mit Hilfe der erfindungsgemäßen Verfahren zugrunde; dabei ist der Einsatz von Ausgestaltungen mit kontinuierlicher Lumineszenzmessung von besonderem Interesse. Beispiele für mögliche Verwendungen im Bereich der Genomanalyse und der Genexpressionsanalyse sind die Nukleinsäuresequenzierung (Pyrosequencing) von DNA oder RNA und der Nachweis oder die Messung von Nukleinsäureamplifikationen durch Polymerase Kettenreaktion (PCR).

Ein weiterer Gegenstand der Erfindung ist eine chemische Zusammensetzung umfassend Dehydro-Luziferin, Luziferin, ATP und Luziferase, die durch Pyrophosphat aktivierbar ist, z.B. Luziferase aus *Photinus pyralis.* Die Zusammensetzung wird, wie oben bereits dargelegt, vorzugsweise als wässrige Lösung ausgestaltet und kann zusätzliche Komponenten enthalten. Die verschiedenen und bevorzugten Möglichkeiten zur Bereitstellung der Zusammensetzung sowie die Lagerstabilität und die weitere Handhabung sind ebenfalls weiter oben bereits erläutert.

Offenbart ist außerdem ein Testset zur Durchführung eines der beschriebenen Verfahren, welches wenigstens
einen geeigneten ersten Behälter, der wenigstens Dehydro-Luziferin enthält,
einen geeigneten zweiten Behälter, der wenigstens Luziferin enthält,
einen geeigneten dritten Behälter, der wenigstens ATP enthält,
einen geeigneten vierten Behälter, der wenigstens Luziferase, die durch Pyrophosphat aktivierbar ist, z.B. Luziferase aus *Photinus pyralis,* enthält, umfasst.

Weitere offenbarte Testsets zur Durchführung eines der beschriebenen Verfahren umfassen wenigstens
einen oder mehrere geeignete Behälter, die jeweils wenigstens eine oder mehrere Komponenten ausgewählt aus der Liste bestehend aus Dehydro-Luziferin, Luziferin, ATP und Luziferase, die durch Pyrophosphat aktivierbar ist, z.B. Luziferase aus *Photinus pyralis,* enthalten, wobei jede gelistete Komponente in wenigstens einem der geeigneten Behälter enthalten ist.

Die Offenbarung betrifft außerdem Testsets zur Durchführung eines der beschriebenen Verfahren, welche wenigstens
einen geeigneten Behälter, der wenigstens Dehydro-Luziferin, Luziferin, ATP und Luziferase, die durch Pyrophosphat aktivierbar ist, z.B. Luziferase aus *Photinus pyralis* enthält,
umfassen.

Die aufgeführten Komponenten können jeweils in Kombination mit zusätzlichen Komponenten vorliegen; Beispiele für zusätzliche Komponenten sind weiter oben bereits dargestellt. Die aufgeführten Komponenten oder Kombinationen von Komponenten liegen, soweit Luziferase nicht umfasst ist, in den geeigneten Behältern vorzugsweise als trockener Feststoff oder trockenes Feststoffgemisch, beispielsweise als Lyophilisat, vor. Wird Luziferase, z.B. Luziferase aus *Photinus pyralis* umfasst, liegen die aufgeführten Komponenten oder Kombinationen von Komponenten vorzugsweise als wässrige Lösung mit einem Glyceringehalt von 10 % bis 60 % Volumenanteil, vorzugsweise 50 % Volumenanteil, vor. Zur Durchführung eines der erfindungsgemäßen Verfahren werden die vorliegenden Komponenten und Kombinationen von Komponenten je nach Ausgestaltung in wässrige Lösung gebracht beziehungsweise wässrig verdünnt, miteinander und mit der Testprobe kombiniert oder in Kontakt gebracht und vermessen.

Geeignete Behälter zeichnen sich durch gute und langfristige Stabilität bei Temperaturen, einschließlich schnellen Temperaturwechseln, im Bereich von plus 20°C bis minus 80°C aus. Bevorzugt sind lichtundurchlässige Behälter mit luft- und wasserdichtem Verschluss aus polymeren Kunststoffmaterialien.

Die offenbarten Testsets können außerdem Beispielprotokolle zur Durchführung unterschiedlicher Ausgestaltungen der erfindungsgemäßen Verfahren, Beispielergebnisse, Referenzproben, Literaturhinweise sowie weitere Materialien enthalten.

Die Erfindung wird durch die nachfolgenden Beispiele näher erläutert:

### Beispiel 1

### Nachweis von Pyrophosphat

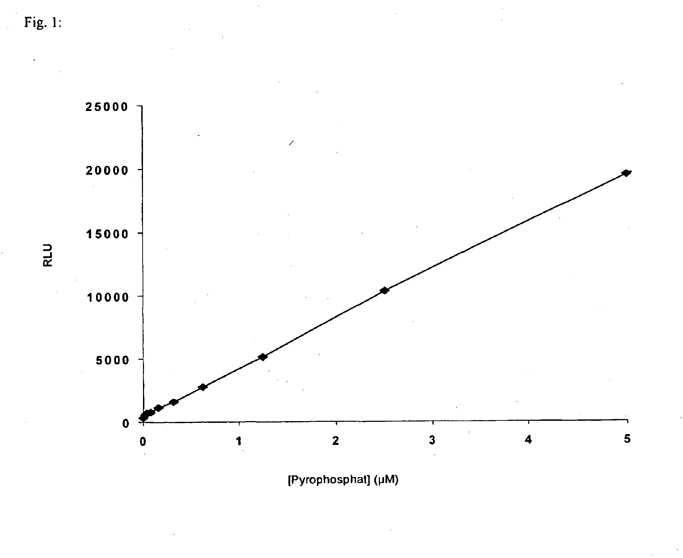

Der Nachweis von Pyrophosphat (PPi) erfolgt nach dem erfindungsgemäßen Verfahren. Das im Test entstehende Signal nimmt mit steigender PPi Konzentration zu (siehe Fig. 1).

### Durchführung des PPi-Nachweises

Zur Durchführung des PPi-Nachweises wurden 40 µL PPi Lösung (Sigma; serielle Verdünnung in Puffer: 50 mM TEA, 2 mM MgCl2, 0,1% BSA (Fraktion V), 0,005% Brij, pH 7,5) in eine Mikroplatte vorgelegt. Anschließend wurden 20 µL Detektionsmix (1,2 nM Firefly Luciferase *(Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in Puffer (50 mM TEA, 2 mM MgCl2, 0,1 % BSA (Fraktion V), 0,005% Brij, pH 7,5) zugegeben. Die Detektionsansätze wurden bei Raumtemperatur luminometrisch vermessen.

### Beispiel 2

### Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des erfindungsgemäßen Verfahrens nachgewiesen. Das im Test entstehende Signal nimmt mit fortschreitender Umsetzung zu und dient als Maß für die sGC-Enzymaktivität. Mit Hilfe einer PPi Referenzkurve (vergleiche Beispiel 1) kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM MgCl2, 0,1% BSA (FraktionV), 0,005% Brij, pH 7,5) in die Mikroplatte vorgelegt und 1 µL der Stimulatorlösung (0-10 µM DEA NONOate (Alexis) in DMSO) hinzugegeben. Der Ansatz wurde 10 min bei Raumtemperatur inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT ( Sigma) in 50 mM TEA, 2 mM MgCl2, 0,1% BSA (Fraktion V), 0,005% Brij, pH 7,5) zugegeben. Die Enzymreaktion wurde durch Zugabe von 20 µl Substratlösung (1,25 mM Guanosine-5'-triphosphat (Sigma) in 50 mM TEA, 2 mM MgCl2, 0,1% BSA (Fraktion V), 0,005% Brij, pH 7,5) gestartet und kontinuierlich luminometrisch vermessen.

### Beispiel 3

### Charakterisierung von Testsubstanzen hinsichtlich Stimulation der sGC Enzymaktivität

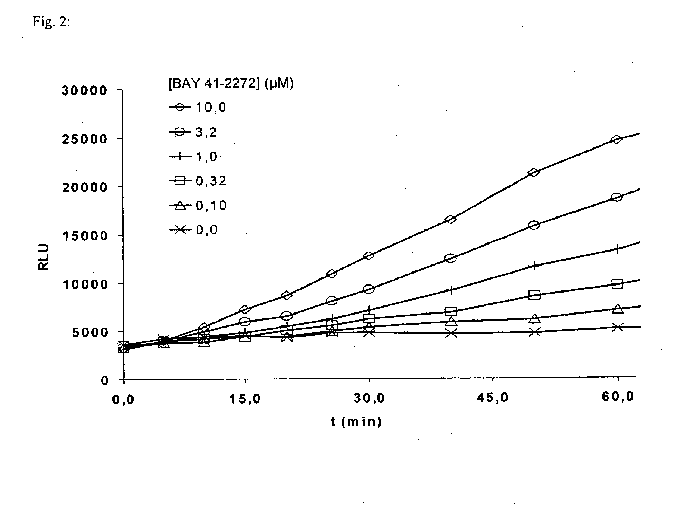

Lösliche Guanylylcyclase (sGC) setzt unter Stimulation GTP zu cGMP und Pyrophosphat (PPi) um. PPi wird mit Hilfe des erfindungsgemäßen Verfahrens nachgewiesen. Das im Test entstehende Signal nimmt mit fortschreitender Umsetzung zu und dient als Maß für die sGC-Enzymaktivität unter der gegebenen Stimulation (siehe Fig. 2).

### Durchführung des Tests:

Zur Durchführung des Tests wurden 29 µL Enzymlösung (0-10 nM lösliche Guanylylcyclase (hergestellt nach Hönicka et al., Journal of Molecular Medicine 77(1999)14-23) in 50 mM TEA, 2 mM MgCl2, 0,1% BSA (FraktionV), 0,005% Brij, pH 7,5) in die Mikroplatte vorgelegt und 1 µL der zu testenden Substanz (z.B. BAY 412272 (Alexis) seriell verdünnt in DMSO) hinzugegeben. Der Ansatz wurde 10 min bei Raumtemperatur inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT ( Sigma) in 50 mM TEA, 2 mM MgCl2, 0,1% BSA (Fraktion V), 0,005% Brij, pH 7,5) zugegeben. Die Enzymreaktion wurde durch Zugabe von 20 µl Substratlösung (1,25 mM Guanosine-5'-triphosphat (Sigma) in 50 mM TEA, 2 mM MgCl2, 0,1% BSA (Fraktion V), 0,005% Brij, pH 7,5) gestartet und kontinuierlich luminometrisch vermessen. Das Maß der Stimulation durch die zu testende Substanz kann relativ zum Signal der nicht stimulierten Reaktion bestimmt werden.

### Beispiel 4

### Vermessung von SQS Enzymaktivität mittels PPi Nachweis

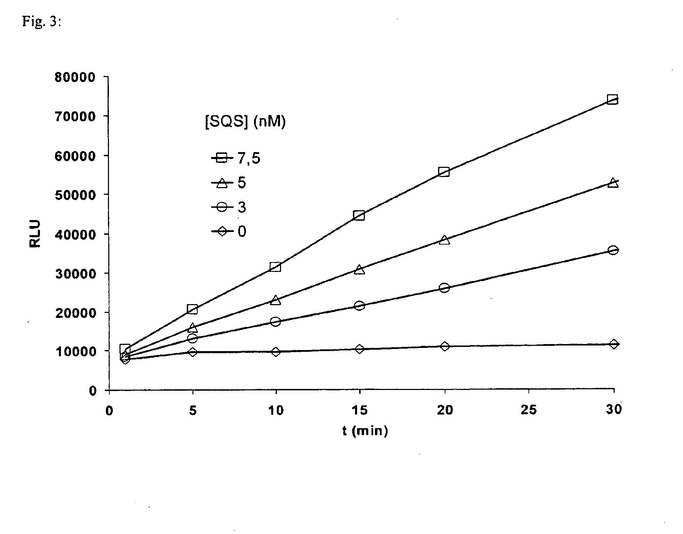

Squalensynthase (SQS) setzt Farnesylpyrophosphat in Gegenwart von NADPH zu Squalen und Pyrophosphat (PPi) um. PPi wird mit Hilfe des erfindungsgemäßen Verfahrens nachgewiesen. Das im Test entstehende Signal nimmt mit fortschreitender Umsetzung zu und dient als Maß für die SQS Enzymaktivität (siehe Fig. 3). Mit Hilfe einer PPi Referenzkurve (vergleiche Beispiel 1) kann das Enzym in bekannter Weise charakterisiert werden, z.B. hinsichtlich Umsatzrate oder Michaelis Konstante.

### Durchführung des Tests:

Zur Durchführung des Tests wurden 40 µl Enzymlösung (0-10 nM SQS (hergestellt nach Soltis, Arch. Biochem. Biophys. 316 (1995) 713) in 50 mM Tris, 5 mM MgCl2, 1,5 mM Glutathion, 5 mM CHAPS, pH 7,5) in die Mikroplatte vorgelegt. Anschließend wurden 20 µl Detektionsmix (1,3 nM Firefly Luciferase (*Photinus pyralis* Luziferase, Promega), 35 µM Dehydro-Luziferin (hergestellt nach Bitler & McElroy, Arch. Biochem. Biophys. 72 (1957) 358), 140 µM Luziferin (Promega), 175 µM ATP (Sigma) und 0,5 mM DTT ( Sigma) in 50 mM Tris, 5 mM MgCl2, 5 mM CHAPS, 0,02% BSA (Fraktion V), pH 7,5) zugegeben. Die Enzymreaktion wurde durch Zugabe von 20 µl Substratlösung (10,5 µM Farnesylpyrophosphat (Sigma), 150 µM NADPH (Sigma) in 50 mM Tris, 5 mM MgCl2, 5 mM CHAPS, pH 7,5) gestartet und kontinuierlich luminometrisch vermessen.

### Beispiel 5

### Charakterisierung von Testsubstanzen hinsichtlich Inhibition der SQS Enzymaktivität

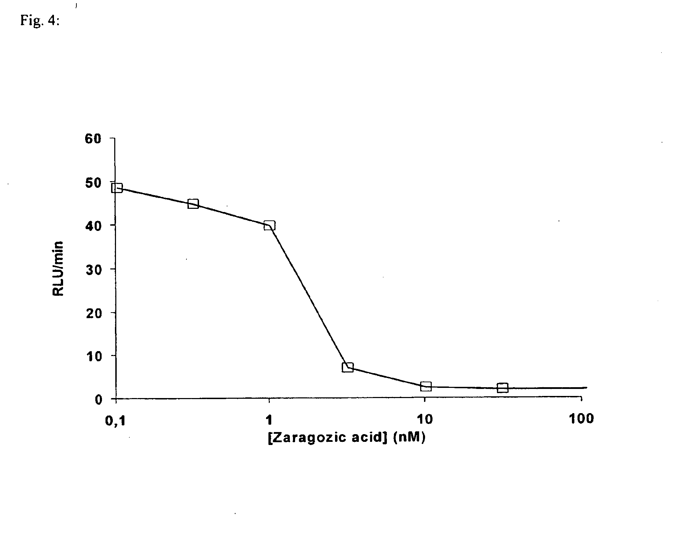

Squalensynthase (SQS) setzt Farnesylpyrophosphat in Gegenwart von NADPH zu Squalen und Pyrophosphat (PPi) um. PPi wird mit Hilfe des erfindungsgemäßen Verfahrens nachgewiesen. Das im Test entstehende Signal nimmt mit fortschreitender Umsetzung zu und dient als Maß für die SQS-Enzymaktivität unter der gegebenen Inhibition (siehe Fig. 4).

### Durchführung des Tests:

Zur Durchführung des Tests wurden 39 µl Enzymlösung (0-10 nM SQS (hergestellt nach Soltis, Arch. Biochem. Biophys. 316 (1995) 713) in 50 mM Tris, 5 mM MgCl2, 1,5 mM Glutathion, 5 mM CHAPS, pH 7,5) in die Mikroplatte vorgelegt und 1 µL der Testsubstanzlösung (serielle Verdünnung in DMSO) hinzugegeben. Der Ansatz wurde 10 min bei Raumtemperatur inkubiert. Anschließend wurden 20 µl Detektionsmix (1,3 nM Firefly Luciferase (*Photinus pyralis* Luziferase, Promega), 35 µM Dehydro-Luziferin (hergestellt nach Bitler & McElroy, Arch. Biochem. Biophys. 72 (1957) 358), 140 µM Luziferin (Promega), 175 µM ATP (Sigma) und 0,5 mM DTT (Sigma) in 50 mM Tris, 5 mM MgCl2, 5 mM CHAPS, 0,02% BSA (Fraktion V), pH 7,5) zugegeben. Die Enzymreaktion wurde durch Zugabe von 20 µl Substratlösung (10,5 µM Farnesylpyrophosphat (Sigma), 150 µM NADPH (Sigma) in 50 mM Tris, 5 mM MgCl2, 5 mM CHAPS, pH 7,5) gestartet und kontinuierlich luminometrisch vermessen. Das Maß der Inhibition kann relativ zum Signal der nicht inhibierten Reaktion bestimmt werden.

Die mögliche Testsubstanz-vermittelte Inhibition der Luziferase Aktivität kann wie folgt überprüft werden:

Nach Abschluss der luminometrischen Messung werden 20 µL einer PPi enthaltenden Kontrolllösung (5 µM PPi (Sigma), 0,02% BSA (Fraktion V, Sigma), 44 µM ATP (Sigma), 35 µM Luziferin (Promega), 0,4 nM Firefly Luciferase (*Photinus pyralis* Luziferase, Promega), 6 µM Dehydro-Luziferin (hergestellt nach Bitler & McElroy, Arch. Biochem. Biophys. 72 (1957) 358) in 50 mM Tris, 2 mM MgCl2, pH 8) zugegeben und erneut luminometrisch vermessen.

Ist der durch Zugabe der PPi enthaltenden Kontrolllösung generierte Signalzuwachs in einem inhibierten Reaktionsansatz so groß wie in einem nicht inhibierten Kontrollansatz, kann eine Inhibition der Luziferasereaktion ausgeschlossen werden.

### Erläuterungen zu den Figuren:

- Fig. 1:: Messung einer Pyrophosphat Konzentrationsreihe; RLU, Lumineszenzsignal in relativen Lumineszenzeinheiten Pyrophosphat], Konzentration Pyrophosphat, mikromolar
- Fig. 2:: Messung der Aktivierung von löslicher Guanylylcyclase (sGC) durch unterschiedliche Konzentrationen von BAY 41-2272; RLU, Lumineszenzsignal in relativen Lumineszenzeinheiten; t, Inkubationszeit in Minuten; [BAY 41-2272], Konzentration BAY 41-2272, mikromolar
- Fig. 3:: Messung der Aktivität von Squalensynthase (SQS); RLU, Lumineszenzsignal in relativen Lumineszenzeinheiten; t, Inkubationszeit in Minuten; [SQS], Konzentration Squalensynthase, nanomolar
- Fig. 4:: Messung der Inhibition von Squalensynthase durch unterschiedliche Konzentrationen von Zaragozic Acid; RLU/min, Veränderung des Lumineszenzsignals pro Zeiteinheit in relativen Lumineszenzeinheiten pro Minute; [Zaragozic Acid], Konzentration Zaragozic Acid, nanomolar

## Patentansprüche

1. Verfahren zum Nachweis von Pyrophosphat, **dadurch gekennzeichnet, dass** es folgende Stufen umfasst:
(a) In Kontakt bringen der Komponenten einer Zusammensetzung umfassend Dehydro-Luziferin, Luziferin, ATP und Luziferase, die durch Pyrophosphat aktivierbar ist, mit der Testprobe
(b) Messung von Lumineszenz.

2. Verfahren nach Anspruch 1, **dadurch gekennzeichnet, dass** die Testprobe aus einer natürlichen Quelle umfassend die belebte und unbelebte Umwelt stammt.

3. Verfahren nach Anspruch 1, **dadurch gekennzeichnet, dass** die Testprobe aus einem pflanzlichen oder tierischen Organismus stammt.

4. Verfahren nach Anspruch 1, **dadurch gekennzeichnet, dass** die Testprobe aus dem menschlichen Körper stammt.

5. Verfahren nach Anspruch 1, **dadurch gekennzeichnet, dass** die Testprobe eine Pyrophosphat bildende oder verbrauchende chemische Reaktion ist.

6. Verfahren nach Anspruch 1, **dadurch gekennzeichnet, dass** die Testprobe eine Pyrophosphat bildende oder verbrauchende Enzymreaktion ist.

7. Verfahren nach Anspruch einen der voranstehenden Ansprüche, **dadurch gekennzeichnet, dass** die Bestimmung der Pyrophosphatkonzentration zeitlich aufgelöst erfolgt.

8. Verfahren nach einem der Ansprüche 6 bis 7, wobei die Enzymreaktion ausgewählt wird aus der Gruppe bestehend aus
Pyrophosphat-bildenden oder Pyrophosphat-verbrauchenden Enzymreaktionen, Guanylylcyclase katalysierten Reaktionen,
Adenylylcyclase katalysierten Reaktionen,
Squalensynthase katalysierten Reaktionen,
Pyrophosphatase katalysierten Reaktionen,
DNA-Polymerase katalysierten Reaktionen oder
RNA-Polymerase katalysierten Reaktionen.

9. Verfahren nach einem der Ansprüche 6 bis 7, **dadurch gekennzeichnet, dass** die Enzymreaktion durch lösliche vaskuläre Guanylylcyclase katalysiert wird.

10. Wässrige Lösung umfassend
Dehydro-Luziferin in einem Konzentrationsbereich von 5 µM bis 250 µM,
Luziferin in einem Konzentrationsbereich von 10 µM bis 500 µM,
ATP in einem Konzentrationsbereich von 10 µM bis 500 µM, und
*Photinus pyralis* Luziferase in einem Konzentrationsbereich von 0,1 nM bis 10 nM.

11. Wässrige Lösung nach Anspruch 10, **dadurch gekennzeichnet, dass** die Photinus pyralis Luziferase eine nicht-rekombinante oder rekombinante Photinus pyralis Luziferase, daraus abgeleitete oder mutierte Photinus pyralis Luziferase oder daraus abgeleitete Mischung ist.

## Claims

1. Method for detecting pyrophosphate, **characterized in that** it includes the following stages:
(a) contacting the components of a composition including dehydroluciferin, luciferin, ATP and luciferase which can be activated by pyrophosphate with the test sample
(b) measuring luminescence.

2. Method according to Claim 1, **characterized in that** the test sample originates from a natural source including the animate and inanimate environment.

3. Method according to Claim 1, **characterized in that** the test sample originates from a vegetable or animal organism.

4. Method according to Claim 1, **characterized in that** the test sample originates from the human body.

5. Method according to Claim 1, **characterized in that** the test sample is a pyrophosphate-forming or - consuming chemical reaction.

6. Method according to Claim 1, **characterized in that** the test sample is a pyrophosphate-forming or - consuming enzyme reaction.

7. Method according to any of the preceding claims, **characterized in that** the determination of the pyrophosphate concentration is effected in a time-resolved manner.

8. Method according to either of Claims 6 and 7, where the enzyme reaction is selected from the group consisting of
pyrophosphate-forming or pyrophosphate-consuming enzyme reactions, guanylyl cyclase-catalyzed reactions,
adenylyl cyclase-catalyzed reactions,
squalene synthase-catalyzed reactions, pyrophosphatase-catalyzed reactions,
DNA polymerase-catalyzed reactions or
RNA polymerase-catalyzed reactions.

9. Method according to either of Claims 6 and 7, **characterized in that** the enzyme reaction is catalyzed by soluble vascular guanylyl cyclase.

10. Aqueous solution including
dehydroluciferin in a concentration range from 5 µM to 250 µM,
luciferin in a concentration range from 10 µM to 500 µM,
ATP in a concentration range from 10 µM to 500 µM, and
*Photinus pyralis* luciferase in a concentration range from 0.1 nM to 10 nM.

11. Aqueous solution according to Claim 10, **characterized in that** the Photinus pyralis luciferase is a non-recombinant or recombinant Photinus pyralis luciferase, a Photinus pyralis luciferase derived or mutated therefrom, or a mixture derived therefrom.

## Revendications

1. Procédé de détection du pyrophosphate, **caractérisé en ce qu'**il comprend les étapes suivantes :
(a) mise en contact, avec l'échantillon d'essai, des composants d'une composition comprenant de la déhydro-luciférine, de la luciférine, de l'ATP et de la luciférase, qui est activée par le pyrophosphate,
(b) mesure de la luminescence.

2. Procédé selon la revendication 1, **caractérisé en ce que** l'échantillon d'essai provient d'une source naturelle comprenant l'environnement animé et non animé.

3. Procédé selon la revendication 1, **caractérisé en ce que** l'échantillon d'essai provient d'un organisme végétal ou animal.

4. Procédé selon la revendication 1, **caractérisé en ce que** l'échantillon d'essai provient du corps humain.

5. Procédé selon la revendication 1, **caractérisé en ce que** l'échantillon d'essai est une réaction chimique formant ou consommant le pyrophosphate.

6. Procédé selon la revendication 1, **caractérisé en ce que** l'échantillon d'essai est une réaction enzymatique formant ou consommant le pyrophosphate.

7. Procédé selon l'une des revendications précédentes, **caractérisé en ce que** la détermination de la concentration du pyrophosphate s'effectue avec une résolution dans le temps.

8. Procédé selon l'une des revendications 6 à 7, dans lequel la réaction enzymatique est choisie dans le groupe consistant en
les réactions enzymatiques formant du pyrophosphate ou consommant du pyrophosphate,
les réactions catalysées par la voie guanylylcyclase,
les réactions catalysées par l'adénylcylcyclase, les réactions catalysées par la squalènesynthase, les réactions catalysées par la pyrophosphatase, les réactions catalysées par l'ADN-polymérase ou les réactions catalysées par l'ARN-polymérase.

9. Procédé selon l'une des revendications 6 à 7, **caractérisé en ce que** la réaction enzymatique est catalysée par une guanylylcyclase vasculaire soluble.

10. Solution aqueuse comprenant :
de la déhydro-luciférine à une concentration comprise dans la plage de 5 à 250 µM,
de la luciférine à une concentration comprise dans la plage de 10 à 500 µM,
de l'ATP à une concentration comprise dans la plage de 10 à 500 µM, et
de la luciférase de *Photinus pyralis,* à une concentration comprise dans la plage de 0,1 à 10 nM.

11. Solution aqueuse selon la revendication 10, **caractérisée en ce que** la luciférase de *Photinus pyralis* est une luciférase de *Photinus pyralis* non recombinante ou recombinante, une luciférase de *Photinus pyralis* dérivée ou mutée à partir de cette dernière, ou un mélange qui en dérive.
